# EUROPÄISCHE PATENTSCHRIFT

(11) **EP 0 356 989 B1**
(45) Veröffentlichungstag und Bekanntmachung des Hinweises auf die Patenterteilung: **06.04.1994**
(21) Anmeldenummer: 89115911.3
(22) Anmeldetag: 29.08.1989
(51) Int. Cl.: C07D 257/06, C07D 257/04, A61K 31/41

(54) **Sulfonamide mit Tetrazolylrest, Verfahren zu ihrer Herstellung sowie Arzneimittel**
Sulfon amides with a tetrazolyl rest, process for their preparation an therapeutic agents
Sulphonamides comportant un reste de tétrazolyle, procédé de préparation et médicaments

(30) Priorität: 31.08.1988 DE 3829431
(43) Veröffentlichungstag der Anmeldung: 07.03.1990
(73) Patentinhaber: BOEHRINGER MANNHEIM GMBH, 68298 Mannheim (DE)
(72) Erfinder: Beckh, Hansjörg, Dr. rer. nat., D-6842 Bürstadt (DE); Witte, Ernst-Christian, Dr. rer. nat., D-6800 Mannheim 1 (DE); Stegmeier, Karlheinz, Dr. rer. nat., D-6148 Heppenheim (DE); Dörge, Liesel, Dr. med., D-6840 Lampertheim (DE)

(56) Entgegenhaltungen:
- EP-A- 0 239 907

## Beschreibung

Die vorliegende Erfindung betrifft Sulfonamide der allgemeinen Formel I 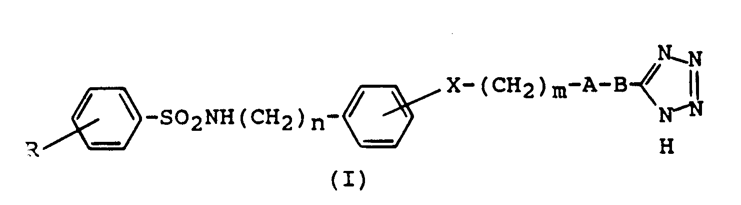
in welcher
- R: Wasserstoff, ein Halogenatom, eine C₁-C₆-Alkyl-, Trifluormethyl- oder Cyanogruppe bedeuten,
- n: eine ganze Zahl von 1 bis 3,
- m: eine ganze Zahl von 0 bis 5 bedeutet,
- X: eine Bindung, Sauerstoff, eine Carbonylgruppe oder eine Gruppe -CHOH-,
- A: eine Bindung oder eine Carbonylgruppe,
- B: eine Bindung oder eine Gruppe -NH- sein kann,
deren physiologisch verträgliche Salze anorganischer oder organischer Basen und Verfahren zu ihrer Herstellung sowie diese Verbindungen enthaltende Arzneimittel.

Für den Fall, daß die Verbindungen der allgemeinen Formel I ein asymmetrisches Kohlenstoffatom enthalten, sind auch die optisch aktiven Verbindungen und racemischen Gemische Gegenstand der Erfindung.

Die Verbindungen der allgemeinen Formel I zeigen eine ausgezeichnete antagonistische Wirkung gegenüber Thromboxan A₂ sowie gegen Prostaglandin-endoperoxide. Sie inhibieren die Aggregation von Blutplättchen und verhindern die Konstriktion der glatten Muskulatur sowie die Bronchokonstriktion. Sie sind außerdem wertvolle Heilmittel zur Behandlung pathologischer Veränderungen der Nierenfunktion.

Diese Eigenschaften machen sie zu wertvollen Heilmitteln zur Behandlung z.B. von cardiovaskulären Erkrankungen und Asthma und zur Prophylaxe einer Schocklunge. Sie können weiterhin verwendet werden bei Organtransplantationen und Nierendialyse und sind geeignet, Rezidive bei Magengeschwüren zu verhindern. Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können z.B. gegen cerebrale ischaemische Zustände eingesetzt werden.

Ist R eine Alkylgruppe, so kann sie geradkettig oder verzweigt sein. Bevorzugt sind Methyl-, Ethyl-, Propyl-, Isopropyl-, Butyl-, sek. Butyl-, tert. Butyl-, Pentyl- und Hexylreste.

Als Halogenatome kommen Fluor, Chlor und Brom in Frage, bevorzugt sind Chlor und Brom. Die Substitution am rechten Phenylring erfolgt vorzugsweise in 3- oder 4-Stellung. Bevorzugt sind Verbindungen mit n = 2.

Das erfindungsgemäße Verfahren zur Herstellung der Verbindungen der Formel I ist dadurch gekennzeichnet, daß man in an sich bekannter Weise entweder
a) eine Verbindung der allgemeinen Formel II 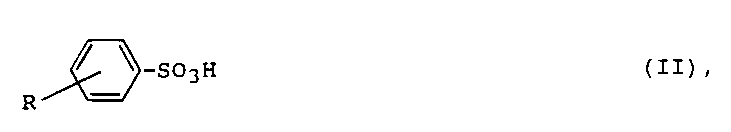 in welcher
   - R: die oben genannte Bedeutung hat, oder ein reaktives Derivat hiervon,
   mit einer Verbindung der allgemeinen Formel III 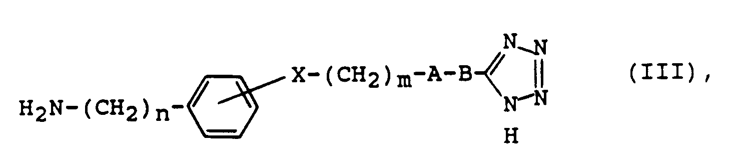
   in welcher A, B, X, n und m die oben genannte Bedeutung haben, oder
b) ein Nitril der allgemeinen Formel IV 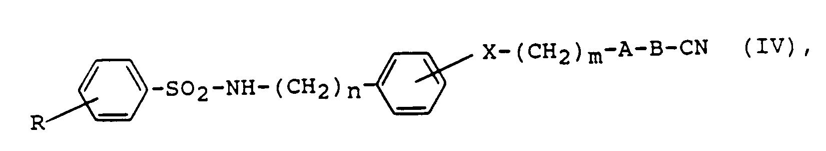 in welcher R, A, B, X, n und m die oben genannte Bedeutung haben,
   mit Stickstoffwasserstoffsäure HN₃ oder einem ihrer Salze reagieren läßt,
   oder
c) für den Fall, daß A eine CO- und B eine -NH-Gruppe darstellt,
   eine Carbonsäure der allgemeinen Formel V 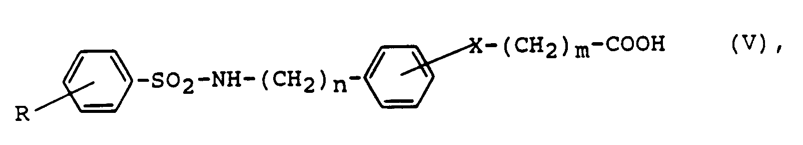 in welcher R, X, n und m die oben genannte Bedeutung haben,
   oder ein reaktives Derivat hiervon, wobei hierunter Ester, wie Methyl-, Cyanmethyl-, Ethyl- und p-Nitrophenylester, Anhydride, Amide wie Imidazolide und Säurehalogenide wie Säurechloride oder Säurebromide verstanden werden,
   mit 5-Amino-1,2,3,4-tetrazol VI, gegebenenfalls als Hydrat, 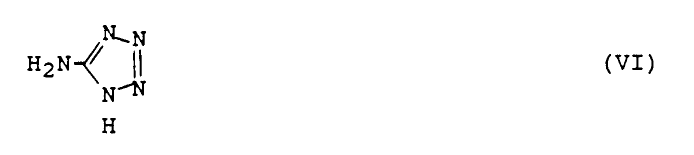 umsetzt.

Ein weiteres Verfahren zur Herstellung von Verbindungen I besteht darin, daß man
d) für den Fall, daß A eine CO- und B eine NH-Gruppe darstellt und m die Zahl Eins bedeutet, eine substituierte Malonsäure der allg. Formel VII 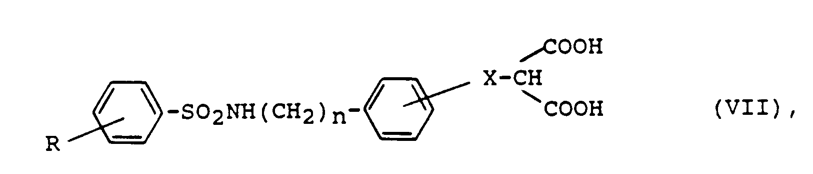 in welcher R und n die oben angegebene Bedeutung haben und X Sauerstoff oder CO symbolisieren,
   oder ein reaktives Derivat einer der beiden Carboxylgruppen mit 5-Amino-1,2,3,4-tetrazol VI, ggf. in Form des Hydrats, umsetzt und anschließend die freie Carboxylgruppe unter Decarboxylierungsbedingungen entfernt,
   und anschließend gewünschtenfalls
   Verbindungen der allgemeinen Formel I in andere Verbindungen der allgemeinen Formel I umwandelt,
   und die erhaltenen Verbindungen der Formel I gewünschtenfalls durch Neutralisation mit nichttoxischen Basen in ihre physiologisch verträglichen Salze umwandelt.

Die Verbindungen der Formel II sind literaturbekannt. Die Verbindungen der Formel III sind zum Teil neu. So können z.B. in an sich bekannter Weise Verbindungen mit X = O, B = NH und A = CO durch Umsetzung eines an der Aminogruppe geschützten Aminoalkylphenols mit einer Halogencarbonsäure und nachfolgender Kondensation der Carboxylgruppe mit 5-Aminotetrazol (Formel VI) erhalten werden.

Sie sind weiterhin zugänglich durch Kondensation eines an der Aminogruppe geschützten Aminoalkylphenols der allg. Formel VIII 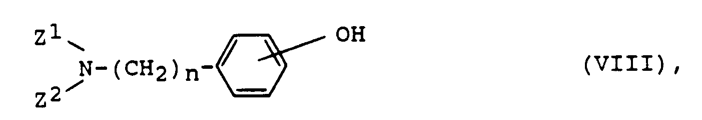
in welcher Z¹Z²N- z.B. einen Benzyloxycarbonylaminorest oder einen Phthaliminorest oder einen Acylamino- oder einen Benzylamino- oder Dibenzylaminorest, aber auch einen Arylsulfonyl-acylamino- oder einen Arylsulfonyl-alkylaminorest darstellen können,
mit einer Verbindung der allgemeinen Formel IX 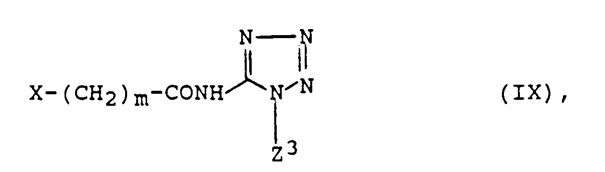
in welcher X eine reaktive Gruppe, bevorzugt ein Halogenatom oder eine Alkyl (Aryl)-sulfonyl-Gruppe darstellt, m die oben angegebene Bedeutung hat und Z³ eine Schutzgruppe, bevorzugt die Benzylgruppe, darstellt,
und ggf. anschließende Entfernung der Schutzgruppen Z¹Z² sowie Z³.

Eine andere Darstellungsmethode geht von Hydroxybenzaldehyden aus: Kondensation eines Hydroxybenzaldehyds mit einer Halogencarbonsäure X

X-(CH₂)m-COOH (X),

und Umsetzen der entsprechenden Verbindung der allg. Formel XI 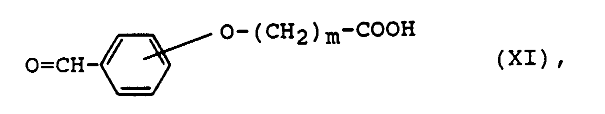
mit 1H-5-Aminotetrazol liefert XII

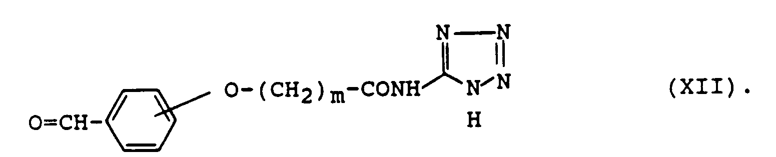

Die Kondensation von XII mit Nitromethan und die Hydrierung/Reduktion des resultierenden Nitrostyrols XIII 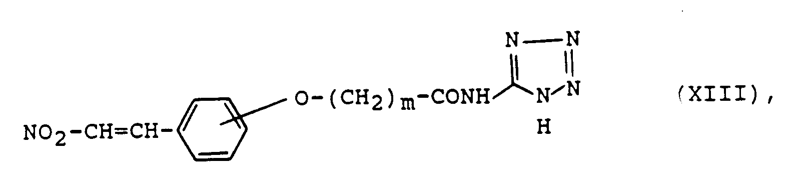
liefert die gewünschten Verbindungen III.

Als Alternative kann auch ein Hydroxynitrostyrol der allg. Formel XIV 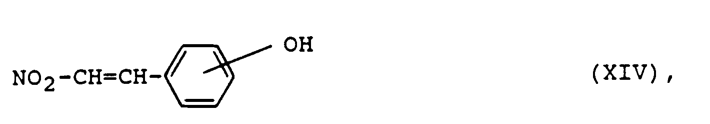
zunächst mit einer Halogencarbonsäure X, dann mit 1H-5-Aminotetrazol umgesetzt werden, wobei wiederum Verbindungen XIII entstehen, die hydriert/reduziert werden müssen.

Auch ist es möglich, ein Nitrostyrol der allg. Formel XIV mit einer Verbindung der allg. Formel IX umzusetzen und anschließend zu hydrieren/reduzieren. Ist Z³ ein Benzylrest, so wird dabei auch dieser entfernt.

Ausgangsmaterial zur Herstellung von Verbindungen III kann auch ein Hydroxy-benzylcyanid der allg. Formel XV sein:

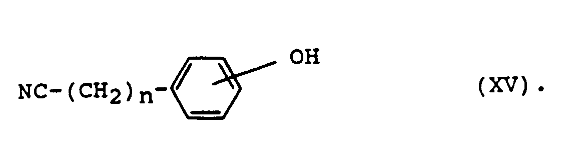

Umsetzen mit einer Halogencarbonsäure X, danach mit 1H-5-Aminotetrazol liefert Verbindungen XVI (R³ = H), Kondensation mit einer Verbindung IX ergibt Verbindungen XVI welche am Stickstoff eine Schutzgruppe Z³ tragen.Anschließend erfolgt eine Reduktion der Nitrilgruppe und ggf. Abspaltung der Schutzgruppe Z³.

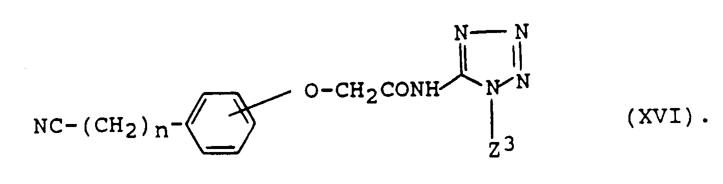

Auch Verbindungen der allgemeinen Formel VII sind neu. Für den Fall, daß X = O bedeutet, erhält man sie durch Umsetzen einer Verbindung XVII 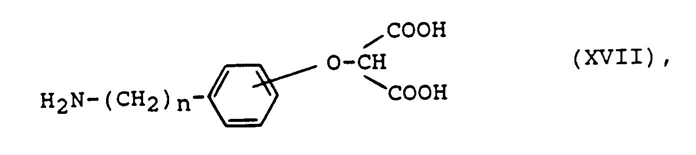
mit einer Sulfonsäure der allg. Formel II.

Die Verbindungen XVII mit n = 2 sind auf mehreren Wegen zugänglich:
Kondensation eines Hydroxy-nitrostyrols XIV mit Chlormalonester in Gegenwart eines Äquivalents Alkali liefert eine Verbindung XVIII 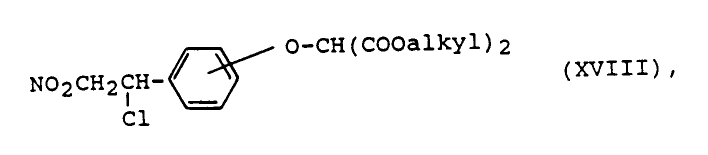
welche durch Hydrieren in den Diester von XVII übergeht.

In Gegenwart von zwei Äquivalenten Alkali entsteht das O-substituierte Nitrostyrol XIX 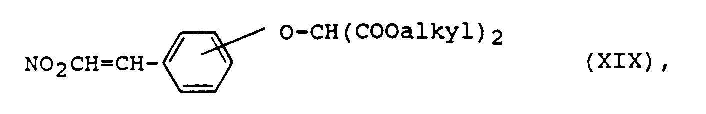
welches man natürlich auch durch Umsetzen eines Hydroxybenzaldehyds mit Chlormalonsäure-diestern zu (XX) 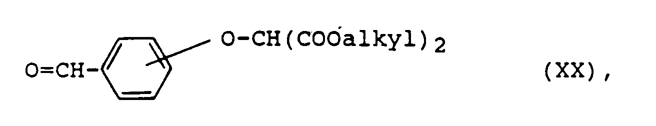
und nachfolgende Kondensation mit Nitromethan erhalten kann. Das Nitrostyrol XIX wird anschließend hydriert/reduziert.

Ohne Einschränkungen hinsichtlich der Zahl n kann als Ausgangsmaterial für Verbindungen XVII ein N-geschütztes Aminoalkylphenol der allgemeinen Formel XXI dienen. 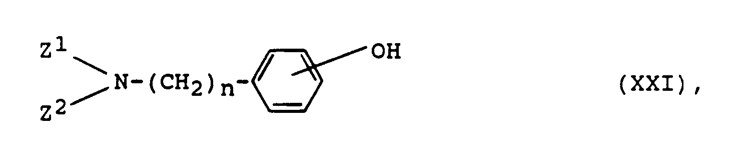
Es wird mit Halogenmalonester kondensiert und anschließend von der Schutzgruppe befreit. Als bevorzugte geschützte Aminogruppen Z¹Z²N- sind hier die Benzyloxycarbonylamino-, die Benzylamino-, die Dibenzylamino- und die Acetylaminogruppe zu nennen.

Bei Verbindungen, in denen A = Valenz bedeutet, geht man von den entsprechenden Nitrilen aus und setzt diese mit Stickstoffwasserstoffsäure zum Tetrazol um.

Für den Fall, daß X = Valenz, A = CO und B = NH bedeuten, geht man von literaturbekannten Phenylalkylcarbonsäuren aus, welche wiederum mit 5-Aminotetrazol kondensiert werden. Für den Fall, daß A = Valenz ist und B = NH bedeutet, werden entsprechende N-Cyan-aralkylamine der Formel XXII 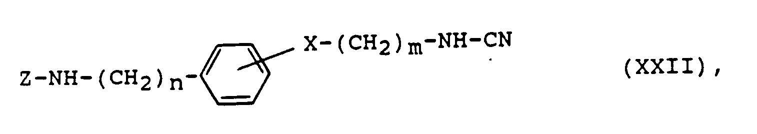
in der
n, m und X die angegebene Bedeutung haben und Z eine Schutzgruppe darstellt
mit Stickstoffwasserstoffsäure zu entsprechenden Aminotetrazol-Derivaten umgesetzt. Die N-Cyanamino-Verbindung erhält man durch Umsetzung des entsprechenden Amins mit BrCN.

Die Umsetzung einer Verbindung der Formel II mit einer Verbindung der Formel III wird zweckmäßig in einem Lösungsmittel oder Lösungsmittelgemisch wie Dichlormethan, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel dienen können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der Formel II, beispielsweise mit deren Anhydrid oder Halogenid, vorzugsweise bei Temperaturen zwischen 0 und 100°C, zum Beispiel bei Temperaturen zwischen Raumtemperatur und 50°C, durchgeführt.

Die Salze der Stickstoffwasserstoffsäure, die in dem erfindungsgemäßen Verfahren mit den Nitrilen IV zur Reaktion gebracht werden, können beispielsweise Alkalimetallsalze sein, wie Lithiumazid, Natriumazid und Kaliumazid, Erdalkalimetallsalze wie Magnesiumazid, Calziumazid und Strontiumazid, oder Metallsalze wie Aluminiumazid, Zinnazid, Zinkazid und Titanazid, Salze mit organischen Basen wie Ammoniumazid und Aniliniumazid und andere. In einigen Fällen ist es zweckmäßig, nicht die reinen Azide einzusetzen, sondern sie im Gemisch z. B. mit Ammoniumchlorid oder mit einem Alkylammoniumchlorid, oder auch in Kombination mit einer Lewis-Säure wie Aluminiumchlorid, Zinnchlorid, Zinkchlorid oder Titantetrachlorid zur Reaktion zu bringen.

In diesem Fall reagieren die Alkalisalze der Stickstoffwasserstoffsäure mit Ammoniumchlorid, Alkylammoniumchlorid oder der Lewis-Säure zum korrespondierenden Salz, wie Ammoniumazid, Alkylammoniumazid, Aluminiumazid, Zinnazid, Zinkazid oder Titanazid, welches mit dem Nitril (IV) reagiert.

Die Kombination der Alkalimetallazide mit Ammoniumchlorid, Alkylammoniumchlorid oder den Lewis-Säuren ergibt besonders gute Ausbeuten.

Die Stickstoffwasserstoffsäure oder ihre Salze sowie die Lewis-Säure oder Ammoniumchlorid bzw. Alkylammoniumchlorid, die in Kombination mit den Alkaliaziden benützt werden, werden im 1-bis 10-molaren Überschuß, bezogen auf die Nitrile (IV), eingesetzt.

Die Reaktion wird zweckmäßig in organischen Lösungsmitteln, beispielsweise Kohlenwasserstoffen, wie z. B. Benzol, Toluol und Petrolether, in Ethern wie z. B. Tetrahydrofuran, Dioxan und Diethylether, oder in aprotisch polaren Solventien, wie z. B. Dimethylformamid, Dimethylsulfoxid oder 1-Methyl-2-pyrrolidinon, durchgeführt.

Die Reaktionsbedingungen wie Temperatur, Druck und Zeit sind keinen besonderen Beschränkungen unterworfen, aber gewöhnlich wird die Reaktion bei Temperaturen zwischen Raumtemperatur und 180°C, bei Drücken zwischen Normaldruck und 160 bar, sowie bei Reaktionszeiten von 30 min bis 48 Stunden, durchgeführt.

Wird ein Salz der Stickstoffwasserstoffsäure eingesetzt, fällt das Produkt wegen der Azidität des Wasserstoffatoms der Tetrazolyl-Gruppe als entsprechendes Salz aus und kann als solches isoliert werden. Durch Behandeln mit einer Mineralsäure wie Salzsäure oder Schwefelsäure erhält man die Verbindung der Formel (I) mit der freien Tetrazolyl-Gruppe.

Die Produkte können in an sich bekannter Weise isoliert und gereinigt werden, beispielsweise durch Fraktionierung entsprechend der Dissoziation des Wasserstoffatoms der Tetrazolyl-Gruppe, durch Chromatographie oder durch Umkristallisation.

Die bevorzugte Methode zur Umsetzung des Aminotetrazols VI mit den Carbonsäuren der allgemeinen Formel V besteht in der Reaktion von etwa äquimolaren Mengen des Amins und der Säure in Gegenwart eines wasserentziehenden Mittels.

Dafür kommt beispielsweise Polyphosphorsäure in Betracht, die dann gleichzeitig als Lösungsmittel dient, oder Chlorameisensäureisobutylester in Gegenwart eines aprotisch polaren Lösungsmittel wie z. B. Dimethylformamid.

Die Reaktionen laufen zwischen 50°C und 200°C ab. Die Endprodukte der allgemeinen Formel I fallen im allgemeinen nach Zugabe von Wasser oder wäßriger Mineralsäure, wie Salzsäure oder Schwefelsäure, aus und werden nach Filtration durch Umkristallisation oder säulenchromatographisch gereinigt.

Eine weitere bevorzugte Methode zur Herstellung von Verbindungen der allgemeinen Formel I besteht in der Umsetzung von etwa äquimolaren Mengen des Amins VI und der Säure V in einem geeigneten Lösungsmittel mit etwa einer äquivalenten Menge eines Halogenierungsmittels, wie Phosphortrichlorid, Phosphorpentachlorid oder Thionylchlorid, bei Temperaturen zwischen Raumtemperatur und der Rückflußtemperatur der Mischung. Geeignete Lösungsmittel sind Methylenchlorid, Tetrachlorkohlenstoff, Diethylether, Toluol, Xylol oder Chlorbenzol. Im allgemeinen fällt das Produkt aus der Lösung aus und wird durch Filtration gewonnen. Falls erforderlich, kann die Reaktionsmischung konzentriert werden bis zu einem Punkt, bei dem das Produkt aus der Lösung auszufallen beginnt. Als weitere Kondensationsmittel bei dieser Reaktion kommen saure Kationenaustauscher, Sulfoniumsalze, Schwefelsäurehalogenide, 2-Halogenpyridiniumsalze, Phosphoniumsalze, N,N′-Dicyclohexylcarbodiimid und Carbonylbisimidazol in Betracht.

Setzt man anstelle der Carbonsäuren ihre Ester ein, so arbeitet man in Gegenwart oder Abwesenheit spezieller Lösungsmittel bei Temperaturen im Bereich von 20°C bis zur Siedehitze des Gemisches. Bevorzugt ist dabei die Reaktion etwa äquimolarer Mengen des Amins und des Esters in Polyphosphorsäure bei 50°C bis 200°C, jedoch kann man auch in einem inerten Lösungsmittel wie Methylenchlorid, Benzol, Toluol, Chlorbenzol am besten in Gegenwart von etwas mehr als einem Äquivalent einer Base wie Natriumethanolat oder Butyllithium oder von Natriumhydrid in Dimethyl-sulfoxid arbeiten.

Setzt man anstelle der Carbonsäure V ihre Anhydride ein, so kann man die Umsetzung mit dem Amin VI schon bei etwas niedrigeren Temperaturen vornehmen. Bevorzugt arbeitet man in einem inerten Lösungsmittel wie Dichlormethan, Diethylether, Benzol, Toluol, bei Temperaturen zwischen Raumtemperatur und 60°C. Man gibt dabei das Amin und das Anhydrid in etwa äquimolaren Mengen zusammen, wobei im allgemeinen eine exotherme Reaktion einsetzt. Nach Abklingen wird zur Vervollständigung der Reaktion noch einige Zeit gelinde erwärmt.

Setzt man anstelle der Carbonsäure ein Säurehalogenid ein, so arbeitet man am besten bei Temperaturen zwischen -10°C und Raumtemperatur. Bevorzugt geht man dabei so vor, daß man nach Schotten-Baumann zur wässrigen Lösung des Amins welche noch eine Base wie Alkalihydroxid, Natriumcarbonat oder Pyridin enthält, das Säurechlorid unter Eiskühlung langsam zutropft und anschließend noch einige Zeit bei Raumtemperatur stehen läßt. Diese Reaktion ist nicht nur in Wasser möglich, sondern auch in organischen Lösungsmitteln wie Methylenchlorid, Ether, Benzol oder Toluol. Auch ohne säurebindende Mittel lassen sich die Amine durch Carbonsäurechloride nahezu quantitativ acylieren, indem man das Amin und das Carbonsäurechlorid in einem inerten Lösungsmittel wie Methylenchlorid,Benzol oder Toluol bis zur Beendigung der Gasentwicklung kocht, was 1 bis 24 Stunden dauert. Gibt man jedoch ein säurebindendes Mittel wie Triethylamin oder Pyridin in geringem Überschuß zu, so läuft die Reaktion schon bei Temperaturen zwischen -10°C und Raumtemperatur ab.

Die Verbindungen der allgemeinen Formel I lassen sich auch dadurch darstellen, daß man die Carbonsäure der allgemeinen Formel V in ihr Imidazolid überführt, zweckmäßigerweise durch Umsetzung der Säure mit N,N-Carbonyldiimidazol in einem organischen Lösungsmittel wie Tetrahydrofuran, Toluol, Benzol oder Diethylether, das Carbonsäure-imidazolid in einen reaktiven Ester umwandelt, und diesen mit dem Tetrazolylamin VI zum Carbonsäure-tetrazolylamid I abreagieren läßt.

Verbindungen der Formel V in denen X = 0 bedeutet, sind u.a. in EP-OS 239 907 beschrieben oder können nach dem dort beschriebenen Verfahren hergestellt werden.

Die Herstellung von Verbindungen I aus Malonsäure der allgemeinen Formel VII erfolgt in ähnlicher Weise:

Man bildet aus VII und einem Mol Carbonylbisimidazol das Monoimidazolid, läßt dieses z.B. mit 4-Nitrophenol zum aktivierten Monoester abreagieren und setzt nun ein Mol 1H-5-Aminotetrazol zu. Anschließend wird auf Temperaturen zwischen 50 und 80°C erhitzt, wobei Amidbildung und Decarboxylierung erfolgen. Als Lösungsmittel verwendet man hier bevorzugt DMF oder DMSO.

Die Kondensation zum Tetrazolamid kann aber auch mit anderen Kondensationsmitteln bzw. anderen Formen der monoaktivierten Malonsäuren VII erfolgen, z.B. mit geeigneten Carbodiimiden.

Die Herstellung der Verbindungen III aus einem geschützten Aminoalkylphenol VIII und einem Halogencarbonsäuretetrazolamid IX erfolgt zweckmäßig in einem Gemisch aus wäßrigem Alkalihydroxid und Ethanol. Andere Reaktionsbedingungen, z.B. die Kondensation mittels Kaliumcarbonat in geeigneten Lösungsmitteln wie z.B. Butanon-2 oder mittels Natriumhydrid in DMF oder DMSO sind ebenfalls geeignet. Die Entfernung der Schutzgruppe R³ = Benzyl erfolgt durch Hydrieren in Alkoholen wie Methanol oder Ethanol in Gegenwart von Chlorwasserstoff (methanolische Salzsäure) an Palladium-Kohle bei Temperaturen zwischen 20 und 80°C und einem Druck bis zu 8 bar.

Verbindungen III erhält man auch durch Umsetzen von Aldehyden der allgemeinen Formel XII mit Nitromethan und nachfolgende Hydrierung/Reduktion des Nitrostyrols XIII. Die Umsetzung von XII mit Nitromethan erfolgt z.B. so, daß man zu einer methanolischen Lösung der Komponenten in der Kälte wäßrige Alkalilauge zutropft. Statt der Alkalilauge kann man vorteilhaft auch Ethylendiammonium-Diacetat als Kondensationsmittel anwenden. Die Hydrierung des Nitrostyrols XIII erfolgt z.B. in Eisessig bei erhöhter Temperatur und einem Wasserstoffdruck bis zu 8 bar in Gegenwart von Palladium-Kohle.

Zur Herstellung von Aminoalkyl-phenoxy-malonsäuren der allgemeinen Formel XVII kondensiert man ein Hydroxy-Nitrostyrol XIV mit Chlor-Malonester und hydriert anschließend. Die Kondensation erfolgt in Gegenwart von Alkalihydroxid in der Kälte. Bevorzugt sind hier die Bedingungen der Phasentransferreaktionen: Reaktion der Komponenten in einem heftig gerührten Gemisch aus wäßrigem Alkali und geeignetem Lösungsmittel, z.B. Methylenchlorid in Gegenwart eines quaternären Ammoniumsalzes, z.B. Tetrabutylammoniumbromid. Sowohl die bei Anwendung eines Mols Alkali resultierenden Chlor-Nitro-Alkyl-Phenoxymalonester XVIII als auch die bei Anwendung von mindestens zwei Mol Alkali resultierenden Nitrostyroloxy-malonsäureester lassen sich zu den Verbindungen XVII hydrieren. Die Hydrierung von XVIII wird entweder zweistufig (zunächst mit Pd/C zur Entfernung des Chloratoms, dann mit Raney-Nickel zur Reduktion der Nitrogruppe) oder, unter Anwendung höheren Drucks und einer höheren Temperatur, einstufig mit Pd/C durchgeführt.

Zur Herstellung von Verbindungen XVII, in denen die Zahl n beliebig sein kann, ist die Umsetzung von N-geschützten Aminoalkylphenolen der allg. Formel XXI mit Halogenmalonester und anschließende Abspaltung der Schutzgruppe besonders bevorzugt. Für die Kondensation von XXI mit Halogenmalonestern eignet sich auch hier besonders die Phasentransfer-Katalyse. Man kann aber auch alle anderen für die Phenyletherbildung geeigneten Verfahren anwenden, also z.B. die Umsetzung mittels K₂CO₃/Butanon-2 oder mittels NaH in DMF oder DMSO. Für den Fall, daß Z¹Z²N- die Bedeutung Benzylamino-, Dibenzylamino-oder Benzyloxycarbonylamino- hat, läßt sich die Schutzgruppe durch Hydrieren entfernen; handelt es sich jedoch um eine Acetylaminogruppe, so folgt eine Hydrolyse mit verd. Alkalilauge.

Die Umwandlung von Verbindungen der allgemeinen Formel I in andere Verbindungen der allgemeinen Formel I kann nach geläufigen Methoden erfolgen.

Steht für A beispielsweise eine Carbonylgruppe, so sind für die Umwandlung in eine Hydroxygruppe alle gängigen Reduktionsverfahren einsetzbar.

Bevorzugt ist die Reduktion mit komplexen Borhydriden, z. B. mit Natriumborhydrid, wobei protische Lösungsmittel wie Wasser, (wässrige) Alkohole oder wässriges Dioxan als Reaktionsmedium dienen. Bei Abwesenheit anderer reduzierbarer Gruppen kann die Reduktion auch mit komplexen Aluminiumhydriden wie LiAlH₄ oder DIBAL (Diisobutylaluminiumhydrid) durchgeführt werden, wobei hier aprotische Lösungsmittel wie Ether, THF oder Dioxan als Reaktionsmedium dienen. Die Carbonylreduktion kann aber auch mit katalytisch angeregtem Wasserstoff erfolgen, z. B. mit H₂/Raney-Nickel oder durch Umsetzen mit Nickel-Aluminium-Legierung in wässrigem Alkali.

Zur Herstellung von Salzen mit physiologisch verträglichen organischen oder anorganischen Basen wie beispielsweise Natriumhydroxid, Kaliumhydroxid, Calciumhydroxid, Ammoniumhydroxid, Methylglucamin, Morpholin, Triethylamin oder Ethanolamin können die Verbindungen der Formel I mit den entsprechenden Basen umgesetzt werden. Auch Mischungen der sauren Verbindungen mit einem geeigneten Alkalicarbonat bzw. - hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Salzen insbesondere die folgenden:
1) 4-[2-(4-Trifluormethyl-benzolsulfonamido)ethyl]phenylessigsäure(1H-tetrazol-5-yl)amid
2) 3-[2-(4-Trifluormethyl-benzolsulfonamido)ethyl]-phenoxyessigsäure(1H-tetrazol-5-yl)amid
3) 3-[2-(4-Chlor-benzolsulfonamido)ethyl]-N-(1H-tetrazol-5-yl)anilin
4) 4-[2-(4-Chlor-benzolsulfonamido)ethyl]-benzyl-N-(1H-tetrazol-5-yl)amin
5) 3-[2-(4-Chlor-benzolsulfonamido)ethyl]-benzyl-N-(1H-tetrazol-5-yl)amin
6) 4-[(4-Chlor-benzolsulfonamido)methyl]phenoxy-essigsäure-(1H-tetrazol-5-yl)amid
7) 3-[(4-Fluor-benzolsulfonamido)methyl]phenoxy-essigsäure-(1H-tetrazol-5-yl)amid
8) 4-[3-(2-Chlor-benzolsulfonylamido)propyl]phenylessigsäure-(1H-tetrazol-5-yl)amid
9) 3-[3-(4-Trifluormethyl-benzolsulfonylamido)propyl]phenylessigsäure-(1H-tetrazol-5-yl)amid
10) 5-<4-[2-(4-Chlor-benzolsulfonamido)ethyl]benzol>-1H-tetrazol
11) 5-<3-[2-(4-Trifluormethyl-benzolsulfonamido)ethyl]benzol>-1H-tetrazol
12) 5-<4-[2-(4-Chlor-benzolsulfonamido)ethyl]benzoyl>-1H-tetrazol
13) 5-<3-[2-(4-Fluor-benzolsulfonamido)ethyl]benzoyl>-1H-tetrazol

### Beispiel 1

### 5-<4-[2-(4-Chlor-benzolsulfonamido)ethyl]phenoxy-methyl>-1H-tetrazol

Man suspendiert 3.50 g (16 mmol) 5-[4-(2-Aminoethyl)phenoxymethyl>-1H-tetrazol und 1.8 g Natronlauge in 100 ml Wasser und gibt 3.55 g (16 mmol) 4-Chlorbenzolsulfonylchlorid bei 40-50°C innerhalb 30 min zu. Dabei wird mittels eines pH-Meters der pH-Wert durch Zutropfen von 5 N Natronlauge bei pH = 10.5 gehalten. Nach einer Stunde Rühren bei 70-80°C wird auf Raumtemperatur abgekühlt, angesäuert und mit Essigester ausgeschüttelt. Die vereinigten organischen Phasen werden nochmals mit verdünnter, wäßriger Natriumhydrogencarbonatlösung extrahiert, angesäuert und mit Essigester ausgeschüttelt. Die organische Phase wird mit Wasser gewaschen, über Natriumsulfat getrocknet und zur Trockne eingedampft. Aus Ethanol erhält man 3.50 g (56 %) farblose Kristalle, Schmp. 136°C.

Das Ausgangsmaterial kann wie folgt erhalten werden:
a) N-(Benzyloxycarbonyl)-p-hydroxyphenethylamin
   34.25 g (0.25 mol) Tyramin werden in 400 ml Wasser gelöst und 35.5. ml (0.25 mol) Chlorameisensäurebenzylester langsam zugetropft. Durch gleichzeitige Zugabe einer konz. Natriumhydrogencarbonat-Lösung wird der pH-Wert mittels pH-Meter kontrolliert auf pH = 9 gehalten.
   Der entstandene Niederschlag wird abgesaugt, mit Wasser gewaschen, in 2 N Natronlauge aufgenommen und dreimal mit Essigester ausgeschüttelt. Die wäßrige Phase wird mit 2 N Salzsäure angesäuert, dreimal mit Essigester extrahiert, über Magnesiumsulfat getrocknet und zur Trockene eingeengt.
   Man erhält 50.30 g (74 %) des N-(Benzyloxycarbonyl)-p-hydroxyphenethyl-amins, farblose Kristalle, Schmp. 100-102°C.
b) 4-[2-(Benzyloxycarbonylamino)ethyl]phenoxyacetonitril
   Man trägt 48.82 g (0.18 mol) des oben dargestellten N-(Benzyloxycarbonyl)-p-hydroxyphenethyl-amins in eine aus 5.17 g Natrium und 270 ml Ethanol frisch dargestellte Natriumethylatlösung (0.23 mol) ein, tropft langsam 11.4 ml (0.18 mol) Chloracetonitril zu und erhitzt zum Sieden.
   Nach 5 Stunden wird das Lösungsmittel abgezogen, der Rückstand in Ether aufgenommen, zweimal mit 2 N Natronlauge ausgeschüttelt, neutral gewaschen, über Natriumsulfat getrocknet und zur Trockne eingeengt. Der ölige Rückstand wird aus Ligroin/Ether (8:2) umkristallisiert. Man erhält 44.74 g (80 %) Produkt in Form farbloser Kristalle, Schmp. 110-115°C.
c) 5-<4-[2-(Benzyloxycarboxyamino)ethyl]phenoxymethyl>-1H-tetrazol
   Man löst 7.60 g (25 mmol) der unter b) dargestellten Verbindung, 5.0 g (77 mmol) Natriumazid und 5.30 g (39 mmol) Triethylamin-Hydrochlorid in 250 ml 1-Methyl-2-pyrrolidinon und rührt bei 150°C 8 Stunden lang. Nach Abkühlen auf Raumtemperatur wird das Lösungsmittel bei 85°C/16 Torr abdestilliert, der ölige Rückstand in 2 N Natronlauge aufgenommen und dreimal mit Ether ausgeschüttelt. Die wäßrige Phase wird mit halbkonzentrierter Salzsäure angesäuert und dreimal mit Essigester extrahiert. Die vereinigten organischen Phasen werden über Magnesiumsulfat getrocknet, das Lösungsmittel abgezogen und der ölige Rückstand mit Ether zur Kristallisation gebracht. Man erhält 8.20 g (93 %) der Titelverbindung, farblose Kristalle, Schmp. 95°C (Z).
d) 5-[4-(2-Aminoethyl)phenoxy]methyl-1H-tetrazol
   8.10 g (23 mmol) des unter c) erhaltenen Tetrazols werden in 400 ml Ethanol gelöst und in Gegenwart von 0.5 g Palladium/Kohle (10proz.) unter Normalbedingungen hydriert.
   Man erhält 4.60 g (91 %) der Titelverbindung, farblose Kristalle, Schmp. 167-168°C.

### Beispiel 2

### 3-[2-(4-Chlor-benzolsulfonamido)ethyl]phenoxyessigsäure(1H-tetrazol-5-yl)amid

In analoger Weise wie in Beispiel 1 beschrieben wird durch Umsetzung des entsprechenden 5-[3-(2-aminoethyl)phenoxy] methyl-tetrazols mit 4-Chlorbenzolsulfochlorid die Titelverbindung erhalten, Schmp. 190-196°C (Zers.; Eisessig)

### Beispiel 3

### 4-[2-(4-Chlor-benzolsulfonamido)ethyl]-N-(1H-tetrazol-5-yl)anilin

Man löst 3.0 g (9 mmol) 4-[2-(4-Chlor-benzolsulfonamido)-ethyl] cyananilid, 2.42 g (45 mmol) Ammoniumchlorid und 2.92 g (45 mmol) Natriumazid in 225 ml trockenem Dimethylformamid und läßt das Gemisch bei 120°C und 150 bar 60 Stunden lang im Autoklaven reagieren. Nach Abdestillieren des Lösungsmittels wird der Rückstand in Essigester aufgenommen und dreimal mit 2 N Natronlauge ausgeschüttelt. Die alkalischen wäßrigen Phasen werden nun mit 2 N Salzsäure angesäuert (Vorsicht, Schutzschild) und dreimal mit Essigester extrahiert. Die vereinigten organischen Phasen werden über Natriumsulfat getrocknet und zur Trockne eingeengt. Der so erhaltene Rückstand wird nochmals in 2 N Natronlauge gelöst, von Unlöslichem abfiltriert und mit 2 N Salzsäure angesäuert. Der entstandene Niederschlag wird abgesaugt und getrocknet. Nach Chromatographie über Kieselgel mit Methylenchlorid/Methanol (9:1) als Laufmittel erhält man die Titelverbindung in Form von schwach rosa Kristallen, Schmp. 148.5-150°C, Ausb. 2.3 g (68 %).

Das Ausgangsmaterial 4-[2-(4-Chlor-benzolsulfonamido)ethyl]-cyananilid kann wie folgt erhalten werden:
a) p-Nitrophenethylamin
   24.2 g (0.20 mol) Phenethylamin werden im Eisbad vorsichtig mit 125 ml konz. Schwefelsäure versetzt. Nach einer Stunde Rühren tropft man bei 0-5°C 16.8 g (0.21 mol) Ammoniumnitrat in 125 ml konz. Schwefelsäure innerhalb 90 min zu. Nach einer weiteren Stunde wird auf 1.5 l Eis gegossen und mit konz. Ammoniumhydroxid auf pH 9 eingestellt. Die wäßrige Phase wird mehrmals mit Ether ausgeschüttelt, die vereinigten organischen Phasen werden über Magnesiumsulfat getrocknet, zur Trockne eingeengt und der Rückstand wird destilliert.
   Man erhält 14.5 g (44 %) eines farblosen Öls, Sdp. 110-115°C/0.01 Torr. ¹H-NMR (60 MHz, CDCl₃, ppm):δ = 2.99 (q, 2H, J = 2.0 Hz, CH₂); 3.05 (t, 2H, J = 2.0 Hz, CH₂).
b) 4-[2-(4-Chlor-benzolsulfonamido)ethyl]-nitro-benzol
   Man löst 14.1 g (85 mmol) des oben erhaltenen p-Nitrophenethylamins und 34 ml (340 mmol) Triethylaminin 170 ml Methylenchlorid und tropft 17.9 g (85 mmol) p-Chlorbenzolsulfonsäurechlorid in 170 ml Methylenchlorid bei 0-5°C innerhalb einer Stunde zu. Nach einer weiteren Stunde Rühren bei 0°C läßt man auf Raumtemperatur kommen. Der Ansatz wird nun auf Eis gegossen, mit 2 N Salzsäure versetzt und mit Methylenchlorid ausgeschüttelt. Die organische Phase wird neutral gewaschen, über Magnesiumsulfat getrocknet und zur Trockne eingeengt. Der ölige Rückstand wird über Kieselgel mit Ligroin/Essigester/Eisessig (1.5:1:0.006) als Laufmittel chromatographiert.
   Man erhält 10.2 g (35 %) des Produkts als farbloses Öl. ¹H-NMR (60 MHz, d₆-DMSO, ppm):δ = 2.7-3.1 (m, 5H, CH₂, NH); 7.35 und 8.10 (AB-System 2d, J = 9.5 Hz, 4H); 7.55 und 7.75 (AB-System, 2d, J = 10 Hz, 4H);
c) 4-[2-(4-Chlor-benzolsulfonamido)ethyl]anilin
   10.0 g (29 mmol) des unter b) erhaltenen Produkts werden in 600 ml Eisessig gelöst, mit 1.5 g Palladium/Kohle (10proz.) versetzt und unter Normaldruck und bei Raumtemperatur hydriert. Nach Filtration wird das Lösungsmittel abgezogen, der Rückstand in Ether aufgenommen und dreimal mit 2 N Salzsäure ausgeschüttelt. Die vereinigten wäßrigen Phasen werden mit 2 N Natronlauge alkalisch gestellt und dreimal mit Essigester extrahiert. Die vereinigten organischen Phasen werden über Magnesiumsulfat getrocknet und zur Trockne eingedampft. Der ölige Rückstand wird in Ether erhitzt und der ausgefallene Niederschlag abgesaugt.
   Man erhält 5.20 g (58 %) der Aminoverbindung in Form farbloser Kristalle, Schmp. 137-140°C.
d) 4-[2-(4-Chlor-benzolsulfonamido)ethyl]-cyananilid
   Zu einer Lösung von 1.71 g (16 mmol) Bromcyan und 4.90 g (16 mmol) des oben erhaltenen 4-[2-(4-Chlorbenzolsulfonamido)ethyl]anilins in 500 ml Toluol gibt man 32 ml (32 mmol) einer 1 N Natriumethylatlösung und rührt 4 Stunden bei Raumtemperatur. Der entstandene Niederschlag wird abfiltriert, mit Toluol gewaschen und die Toluol-Phase zur Trockne eingeengt.
   Der ölige Rückstand wird nun mit dest. Wasser versetzt, mit 10 N Natronlauge alkalisch gestellt, der entstandene Niederschlag scharf abgesaugt und mit Wasser gewaschen. Aus Ethanol erhält man 3.40 g (63 %) des Produkts in Form schwach gelber Kristalle, Schmp. 137-140°C.

### Beispiel 4

### 4-[2-(4-Chlor-benzolsulfonamido)ethyl]phenoxyessigsäure-(1H-tetrazol-5-yl)amid

Zu einer Suspension aus 7.30 g (20 mmol) 4-[2-(4-Chlorbenzolsulfonamido)ethyl]phenoxyessigsäure (Darstellung s. EP-OS 239 907) und 2.20 ml 4-Methylmorpholin in 60 ml trockenem Methylenchlorid tropft man bei -10°C 2.76 g (20 mmol) Chlorameisensäureisobutylester, gelöst in 40 ml trockenem Methylenchlorid, innerhalb 15 Minuten zu. Nach weiteren 15 Minuten wird eine Lösung aus 2.06 g (20 mmol) 5-Amino-1,2,3,4-tetrazol und 20 ml Dimethylformamid innerhalb 15 Minuten zugetropft. Nach 1 Stunde Rühren bei -10°C läßt man über Nacht auf Raumtemperatur kommen, destilliert die Lösungsmittel ab, nimmt den öligen Rückstand in Essigester auf und schüttelt mit 2 N Natronlauge aus. Die alkalischen wäßrigen Phasen werden mit 2 N Schwefelsäure angesäuert, der entstandene Niederschlag abgesaugt und mit Wasser gründlich gewaschen. Aus Ethanol erhält man 4.20 g (48 %) farbloser Kristalle, Schmp. 227.5-229.5°C.

### Beispiel 5

### 4-[2-(4-Chlor-benzolsulfonamido)ethyl]phenylessigsäure-(1H-tetrazol-5-yl)amid

Zu einer Suspension aus 2.0 g (5 mmol) 4-[2-(4-Chlorbenzolsulfonamido)ethyl]phenylessigsäure-imidazolid in 5 ml Dimethylsulfoxid gibt man 0.7 g (5 mmol) 4-Nitrophenol, rührt 10 Minuten lang und erwärmt dabei den Ansatz, bis sich eine klare Lösung gebildet hat. Zu dieser gibt man 0.425 g (5 mmol) 5-Amino-tetrazol, rührt 30 Minuten bei 90°C, kühlt auf Raumtemperatur ab und gießt nun den Ansatz auf Wasser. Der ausgefallene Niederschlag wird abfiltriert, mit Wasser gewaschen und aus Ethanol umkristallisiert. Man erhält 1.50 g (72 %) der Titelverbindung in Form farbloser Kristalle, Schmp. 248°C.
Die Ausgangsverbindung 4-[2-(4-Chlor-benzolsulfonamido)ethyl]phenylessigsäure-imidazolid erhält man wie folgt:
Man versetzt 7.10 g (20 mmol) 4-[2-(4-Chlor-benzolsulfonamido)ethyl]phenylessigsäure in 50 ml THF bei 50°C mit 3.25 g (20 mmol) N,N-Carbonyldiimidazol, saugt den ausgefallenen Niederschlag nach 15 Minuten ab und wäscht ihn mit Ether. Man erhält 5.30 g (66 %) des Produkts in Form farbloser Kristalle, Schmp. 168-170°C.

### Beispiel 6

I) 4-Oxo-4-<4-[2-(benzolsulfonamido)ethyl]phenyl>buttersäure-(1H-tetrazol-5-yl)amid
   Analog Beispiel 3 erhält man aus 7.20 g (20 mmol) 4-Oxo-4-<4-[2-(benzolsulfonamido)ethyl]phenyl>buttersäure und 4.11 g (40 mmol) 5-Amino-1,2,3,4-tetrazol 2.20 g (26 %) der Titelverbindung, farblose Kristalle, Schmp. 241-242°C. Entsprechend wurden dargestellt:
II) 4-Oxo-4-<4-[2-(4-chlor-benzolsulfonamido)ethyl]phenyl>buttersäure-(1H-tetrazol-5-yl)amid
   Ausbeute: 75 %, farblose Kristalle, Schmp. 238-239°C (DMF/H₂O);
III) 4-Oxo-4-<4-[2-(4-brom-benzolsulfonamido)ethyl]phenyl>buttersäure-(1H-tetrazol-5-yl)amid
   Ausbeute: 67 %, farblose Kristalle, Schmp. 239-239.5°C (DMF/H₂O);
IV) 4-Oxo-4-<4-[2-(4-cyano-benzolsulfonamido)ethyl]phenyl>buttersäure-(1H-tetrazol-5-yl)amid
   Ausbeute: 59 %, farblose Kristalle, Schmp. 238-239°C (DMF/H₂O)
V) 4-Oxo-4-<4-[2-(p-tolylsulfonamido)ethyl]phenyl>buttersäure-(1H-tetrazol-5-yl)amid
   Ausbeute: 63 %, farblose Kristalle, Schmp. 222-223°C (Methanol)
   Das Ausgangsmaterial 4-Oxo-4-<4-[2-(4-R-benzolsulfonamido)ethyl]phenyl>buttersäure läßt sich wie folgt darstellen:

a) N-Acetylphenethylamin
   Zu einer Lösung von 376 ml (3.0 mol) Phenethylamin in 1.5 L Toluol tropft man 350 ml (3.14 mol) Essigsäureanhydrid innerhalb 50 min zu, die Temperatur steigt dabei auf 68°C. Man erhitzt 1 h zum Rückfluß, dampft das Toluol im Hochvakuum ab und destilliert den Rückstand bei 1.5 mm Hg.
   Ausbeute: 475 g (97 %), Sdp. 162-166°C/1.5 mm Hg, Schmp. 50-52°C.
b) 4-Oxo-4-<4-[2-(N-acetylamino)ethyl]phenyl>buttersäuremethylester
   Zu einer Lösung von 110.97 g (0.68 mol) N-Acetylphenethylamin und 93 ml Bernsteinsäuremethylesterchlorid (0.75 mol) in 1200 ml abs. Methylenchlorid trägt man bei 0°C portionsweise 272.7 g (2.04 mol) AlCl₃ ein, rührt noch eine Stunde bei 0°C und erwärmt den Ansatz auf Raumtemperatur. Man gießt auf HCl/Eiswasser, trennt die organische Phase ab, trocknet über MgSO₄ und dampft eine. Den Rückstand rührt man mit heißem Ether aus, saugt ab und wäscht mit wenig kaltem Ether nach.
   Ausb. 154 g (82 %) farblose Kristalle, Schmp. 119.5°C.
c) 4-Oxo-4-[4-(aminoethyl)phenyl)buttersäure
   Man erhitzt 130 g (0.47 mol) der Verbindung aus b) in 1200 ml 6 N HCl 8 Stunden zum Rückfluß, kühlt auf 0°C ab und saugt den erhaltenen Niederschlag ab.
   Ausbeute: 103.1 g (99.6 %) farblose Kristalle, Schmp. 234-235°C (Ethanol).
d) 4-Oxo-4-<4-[2-(4-R-benzolsulfonamido)ethyl]phenyl>buttersäure
   Zu 8.85 g (40 mmol) der Verbindung aus c) in 220 ml 2 N NaOH gibt man 40 mmol 4-R-Benzolsulfonsäurechlorid innerhalb 30 Minuten zu und hält den pH-Wert durch Zutropfen von 5 N NaOH bei pH 10.5. Nach beendeter Zugabe rührt man noch eine Stunde bei 70-80°C, kühlt ab und schüttelt mit Ether aus. Die wässrige Phase stellt man dann auf pH 4-5 ein, extrahiert dreimal mit Essigester, trocknet die organischen Phasen über MgSO₄ und destilliert das Lösungsmittel ab. Der Rückstand wird aus Ether umkristallisiert.

1) R = H: Ausbeute: 9.9 g (69 %), farblose Kristalle, Schmp. 156-158°C
2) R = Cl: Ausbeute: 11.1 g (70 %), farblose Kristalle, Schmp. 151-151.5°C
3) R = Br: Ausbeute: 10.3 g (59 %), farblose Kristalle, Schmp. 150-152°C
4) R = CN: Ausbeute: 10.73 g (69 %), farblose Kristalle, Schmp. 148-150°C
   Das p-Cyano-benzolsulfonsäurechlorid wurde durch Umsetzung von 4-Sulfonylbenzoesäure mit PCl₅ (J.pharm.Soc.Japan 69, 417 (1949); C.A. 1950, 1924) gewonnen.
5) R = CH₃: Ausbeute: 11.6 g (77 %) schwache gelbe Kristalle, Schmp. 133-135°C

### Beispiel 7

### 4-Hydroxy-4-<4-[2-(4-chlor-benzolsulfonamido)ethyl]phenyl>buttersäure-(1H-tetrazol-5-yl)amid

Zu einer Suspension von 3.7 g (80 mmol) der Ketoverbindung aus Beispiel 6 II) in 80 ml 0.6proz. Natronlauge tropft man bei 0°C eine Lösung von 0.30 g NaBH₄ in 3.2 ml 0.2 N Natronlauge (80 mmol) innerhalb von 15 Minuten zu. Man rührt den Ansatz 30 Minuten bei Raumtemperatur, dann 2 Stunden bei 60°C, kühlt ab und wäscht mit Ether. Die wässrige Phase säuert man mit 5 N HCl an, extrahiert mit Essigester, trocknet über Magnesiumsulfat und engt zur Trockne eine.

Der Rückstand wird aus DMF/H₂O umkristallisiert. Man erhält 2.8 g (75 %) der Titelverbindung, farblose Kristalle vom Schmp. 194.5-195.5°C.

### Beispiel 8

### 5-<4-[2-(4-Chlor-benzolsulfonamido)ethyl]phenylmethyl>-1H-tetrazol

Man erhitzt 4.62 g (13.8 mmol) 4-[2-(4-Chlor-benzolsulfonamido)ethyl]phenylacetonitril, 2.94 g (21.3 mmol) Triethylammoniumchlorid und 2.77 g (42.6 mmol) Natriumazid in 150 ml 1-Methylpyrrolidon 6 Stunden auf 150°C. Das 1-Methylpyrrolidon wird anschließend im Hochvakuum bei 80-90°C abdestilliert, der Rückstand in verdünnter Natronlauge gelöst und dreimal mit Essigester ausgeschüttelt. Die wässrige Phase säuert man mit HCl an, extrahiert ebenfalls dreimal mit Essigester, trocknet über Natriumsulfat und dampft nach Behandlung mit Aktivkohle zu Trockne ein.

Der Rückstand wird in NaHCO₃-Lösung aufgenommen, mit Essigester ausgeschüttelt und angesäuert, dabei kristallisiert das Produkt aus. Man erhält 1.5 g (29 %) der Titelverbindung, farblose Kristalle, Schmp. 168-169°C.

Das Ausgangsmaterial 4-[2-(4-Chlor-benzolsulfonamido)ethyl]phenylacetonitril erhält man wie folgt:
a) 4-[2-(4-Chlor-benzolsulfonamido)ethyl]benzoesäureethylester
   Man rührt eine Suspension von 11.5 g (50 mmol) 4-(2-Aminoethyl)benzoesäureethylester-Hydrochlorid (J.Am.Chem.Soc. 65, 2281, 2284 (1943)) und 12.65 g (125 mmol) Triethylamin in 150 ml Methylenchlorid 30 Minuten bei Raumtemperatur, kühlt auf 0°C ab und tropft eine Lösung von 11.55 g (50 mmol) 4-Chlorbenzolsulfonsäurechlorid in 50 ml Methylenchlorid langsam zu. Nach Beendigung der Reaktion schüttelt man zweimal mit verd. HCl aus, wäscht mit Wasser und trocknet die organische Phase über Natriumsulfat. Man erhält nach Abdampfen des Lösungsmittels 11.4 g (62 %) farblose Kristalle, Schmp. 88°C (Ethanol).
b) 4-[2-(4-Chlor-benzolsulfonamido)ethyl]benzyl-alkohol
   Zu einer Suspension von 11.0 g (30 mmol) der Benzoesäure in 100 ml abs. THF tropft man bei Raumtemperatur 1.2 g (30 mmol) LiAlH₄ in 100 ml abs. THF und erhitzt zwei Stunden zum Rückfluß. Der Ansatz wird anschließend mit Eiswasser/2 N H₂SO₄ hydrolysiert und der ausfallende Niederschlag abgesaugt. Die Ausbeute beträgt 7.0 g (72 %), farblose Kristalle vom Schmp. 145-146°C (Ethanol).
c) 4-[2-(4-Chlor-benzolsulfonamido)ethyl]benzylchlorid
   Man löst 5.5 g (16.9 mmol) des Benzylalkohols in 30 ml Benzol, fügt 3.0 g (25 mmol) Thionylchlorid und 5 Tropfen Pyridin hinzu und erhitzt 30 Minuten zum Rückfluß. Der Ansatz wird zur Trockne eingedampft und der Rückstand aus Isohexan/Ether umkristallisiert. Die Ausbeute beträgt 5.6 g (95 %) farblose Kristalle, Schmp. 96-98°C.
d) 4-[2-(4-Chlor-benzolsulfonamido)ethyl]benzylcyanid
   Man löst 5.43 g (15.8 mmol) des Benzylchlorids und 1.18 g (24 mmol) Natriumcyanid in 40 ml 90proz. Ethanol und rührt den Ansatz zwei Stunden bei 80°C. Das Lösungsmittel wird nun abdestilliert, der Rückstand mit Wasser versetzt und mit Methylenchlorid extrahiert. Man erhält 5.2 g (99 %) eines farblosen Öls.
   ¹H-NMR (300 MHz, d₆-DMSO, ppm) δ = 2.70 (t, J =6 H_{z}, 2H, CH₂); 3.00 (q, J = 6 Hz, 2H, CH₂); 3.25 (s, 2H, CH₂-CN); 7.15 und 7.23 (2d, AA′BB′-System, J = 9 Hz, 4H, p-Phenylen); 7.62 und 7.76 (2d, AA′BB′-System, J = 10 Hz, 4 H, p-Chlorphenylen);
   IR (cm⁻¹): V_{N-H} = 3249 (s); V_{C-N} = 2200 (w);

### Beispiel 9

### 5-<4-[2-(4-Chlor-benzolsulfonamido)ethyl]phenyl>pentansäure-(1H-tetrazol-5-yl)amid

Die Darstellung erfolgt analog Beispiel 5:
Zu einer Lösung von 7.0 g (17.7 mmol) 5-<4-[2-(4-Chlorbenzollsulfonamido)ethyl]phenyl>pentansäure in 100 ml abs. THF gibt man portionsweise 2.9 g (17.7 mmol) Carbonyldiimidazol, dann 2.5 g (17.7 mmol) Nitrophenol und nach 10 Minuten 1.5 g (17.7 mmol) Aminotetrazol. Man erhitzt zwei Stunden auf 60°C, dampft das THF ab und verrührt den Rückstand mit Wasser. Der Niederschlag wird abgesaugt und aus Ethanol umkristallisiert.
Ausbeute: 3.7 g (45 %) farblose Kristalle, Schmp. 226°C.

Die Vorstufe 5-<4-[2-(4-Chlor-benzolsulfonamido)ethyl]phenyl>pentansäure erhält man wie folgt:
a) 5-Oxo-5-[4-(2-Acetaminoethyl)phenyl]pentansäuremethylester (vgl. C.A. 54, 22474e)
   Zu einer Lösung von 26.9 g (0.165 mol) Acetylphenethylamin (Darstellung s. J.Am.Pharm.Assoc. 47, 353 (1958) in 300 ml Methylenchlorid tropft man zunächst 30.0 g (0.182 mol) Glutarsäuremethylesterchlorid und gibt dann bei 0-5°C 66.1 g (0.495 mol) AlCl₃ portionsweise zu. Man rührt noch eine Stunde bei 0°C, läßt auf Raumtemperatur kommen und gießt den Ansatz nach vier Stunden auf Eis/HCl. Die organische Phase wird abgetrennt, mit Wasser neutral gewaschen und über Natriumsulfat getrocknet. Man erhält nach Abziehen des Lösungsmittels 34 g (71 %) farblose Kristalle, Schmp. 107-108°C (Essigester).
b) 5-[4-(2-Acetaminoethyl)phenyl]pentansäuremethylester
   In einer Hydrierapparatur werden 9.0 g (31 mmol) des Ketons in 100 ml Methanol gelöst und bei Raumtemperatur und 5 bar Druck hydriert. Als Katalysator verwendet man 1.0 g 10proz. Pd/C und 1 ml conc. HCl. Nach Aufnahme von 1.2 l H₂ wird abgebrochen, der Katalysator abgesaugt und das Methanol abdestilliert. Der Rückstand wird in Ether aufgenommen, mit 2 N NaHCO₃ gewaschen, über Natriumsulfat getrocknet und eingedampft. Man erhält 6.6 g (77 %) farblose Kristalle, Schmp. 57-59°C (Ligroin).
c) 5-[4-(2-Aminoethyl)phenyl]pentansäureethylester
   Man erhitzt eine Lösung von 6.4 g (23.1 mmol) der Acetamino-Verbindung in 40 ml 6 N HCl 8 Stunden zum Rückfluß. Beim Abkühlen fällt 5-[4-(2-Aminoethyl)phenyl]pentansäure als Hydrochlorid aus; es wird abgesaugt, mit 2 N HCl nachgewaschen und über KOH getrocknet. Man erhält 2.8 g (47 %) farblose Kristalle, Schmp. 210-213°C.
   ¹H-NMR (300 MHz, d₆-DMSO, ppm, nach Schütteln mit D₂O): δ = 1.20 (t, J = 6.1 Hz, 3H, CH₃); 1.55 (m, 4H, CH₂-CH₂); 2.30 (t, J = 7 Hz, 2H, CH₂-COOEt); 2.55 (t, J = 6 Hz, 2H, Ph-CH₂); 2.90 (q, J = 6 Hz, 2H, CH₂); 3.05 (t, J = 9 Hz, 2H, Ph-CH₂); 4.05 (q, J = 6.5 Hz, 2H, CH₂); 7.15 und 7.17 (2d, J = 8 Hz, AA′BB′-System 4H, p-Phenylen);
   Zur Überführung in den Ethylester löst man 2.7 g (10.5 mmol) in 100 ml Ethanol und leitet etwa 2 Stunden lang HCl-Gas durch die Lösung. Das Lösungsmittel wird abdestilliert, der Rückstand mit eiskalter, verdünnter NaOH versetzt und mit Ether extrahiert. Die organische Phase wird nochmals mit Wasser gewaschen über Natriumsulfat getrocknet und eingedampft. Man erhält 2.4 g (92 %) der freien Base (farbloses Öl). Sie ist unbeständig und muß schnell weiterverarbeitet werden.
   IR (cm⁻¹): V_{C-O} = 1730 (s); V_{C-H} = 2840-3000 (s) V_{N-H} = 3350 (m);
d) 5-<4-[2-(4-Chlor-benzolsulfonamido)ethyl]phenyl>pentansäure
   Zu einer Suspension von 6.0 g (21 mmol) der Aminopentansäure und 5.3 g (52.5 mmol) Triethylamin in 100 ml Methylenchlorid gibt man bei 0°C portionsweise 4.4 g (21 mmol) 4-Chlorbenzolsulfonsäurechlorid zu und rührt noch je eine Stunde bei 0°C und bei Raumtemperatur. Der Ansatz wird mit 2 N HCl ausgeschüttelt, die organische Phase über Natriumsulfat getrocknet und eingedampft. Man erhält 9.8 g des Esters als schwach gelbes Öl, das durch Erhitzen mit 30 ml 2 N NaOH in 30 ml Methanol auf 50°C und anschließendem ansäuern mit konz. HCl in die freie Säure überführt wird. Die Ausbeute beträgt 7.4 g (85 %), farblose Kristalle, Schmp. 118-120°C.

### Beispiel 10

### 3-[2-(4-Chlor-benzolsulfonamido)ethyl]phenoxyessigsäure-(1H-tetrazol-5-yl)amid

Zu einer Lösung aus 300 ml abs. THF und 2.07 g (5 mmol) der nach 10 e) erhaltenen Malonsäure gibt man 1.03 g (5 mmol) Dicyclohexylcarbodiimid, rührt 10 Minuten und fügt dann 0.51 g (5 mmol) 5-Amino-1H-tetrazolhydrat zu. Anschließend wird drei Stunden bei 50°C gerührt, i. Vak. eingedampft und zum Rückstand Soda gegeben (10 mmol in Form einer 2 N Na₂CO₃-Lösung. Man verrührt 10 Minuten lang, filtriert Dicyclohexylharnstoff ab und säuert das Filtrat an. Nach dem Absaugen wird getrocknet und aus 95proz. Ethanol umkristallisiert.
Ausb. 1.89 g (87 %), Schmp. 184-186°C.

Die Verbindung kann auch nach folgendem Verfahren erhalten werden:
In eine 45°C warme Lösung aus 200 ml DMSO und 2.07 g (5 mmol) der nach 10 e) erhaltenen Malonsäure gibt man 0.81 g (5 mmol) Carbonylbisimidazol, rührt 10 Minuten, trägt 0.70 g (5 mmol) 4-Nitrophenol und 0.425 g (5 mmol) wasserfreies 5-Amino-1H-tetrazol ein und hält nun zwei Stunden auf 80°C. Danach wird abgekühlt, in 2 N HCl eingerührt, abgesaugt und nach dem Trocknen aus 95proz. Ethanol umkristallisiert.
Ausb. 1.55 g (71 %), Schmp. 186-187°C.

Die Ausgangsverbindung <3-[2-(4-Chlor-benzolsulfonamido)ethyl]phenoxy>malonsäure wird auf folgendem Wege erhalten:
a) 3-(2-Benzylamino-ethyl)phenol
   Man rührt ein Gemisch aus 17.6 g (79.6 mmol) Tetrahydropyran-2-ylether des 3-(2-Aminoethyl)phenols, 150 ml Ethanol und 8.9 g (83.5 mmol) Benzaldehyd eine Stunde lang bei Raumtemperatur, gibt unter heftigem Rühren bei 0°C 3.5 g (92 mmol) NaBH₄ zu und läßt eine Stunde bei 0°C und eine Stunde bei Raumtemperatur nachreagieren. Dann wird i. Vak. eingedampft und der Rückstand mit Ether und Wasser versetzt. Nach Durchschütteln wird die Etherphase abgetrennt, mit Na₂SO₄ getrocknet und tropfenweise mit konz. H₂SO₄ versetzt. Das ausgefallene Sulfat löst man in Wasser, gibt etwas 2 N HCl zu, erhitzt 10 Minuten auf dem Wasserbad und kühlt ab. Durch Zugabe von Soda wird die Base in Freiheit gesetzt. Man extrahiert sie mit Ether, trocknet die etherische Lösung (Na₂SO₄) und dampft ein.
   Ausb. 15.0 g (83 %), Schmp 86-88°C.
   ¹H-NMR (300 MHz, d₆-DMSO, ppm):δ = 2.62 - 2.75 (m, 4H, CH₂CH₂).
b) [3-(2-Benzylamino-ethyl)phenoxy]malonsäure
   Zu einer Lösung aus 8.0 g (35.2 mmol) 3-(2-Benzylaminoethyl)phenol und 100 ml Ethanol gibt man 35.2 mmol Natriummethylat in Form einer methanol. Lösung und danach 6.8 g (35.2 mmol) Chlormalonsäurediethylester. Nach 4 Stunden Rühren wird eingedampft. Zum Rückstand gibt man 50 ml Ether und eiskalte 1 N NaOH, schüttelt durch und trennt die Etherphase ab. Nach Trocknen (Na₂SO₄) fällt man aus ihr durch tropfenweise Zugabe von konz. H₂SO₄ das Sulfat aus, saugt ab und wäscht mit Ether. Das Produkt ist sehr hygroskopisch. Etwas schmierige Masse.
   Ausb. 11.7 g (74 %)
   ¹H-NMR (300 MHz, d₆-DMSO, ppm): = 2.90 - 2.98 und 3.11. - 3.22 (2 m, je 2H, CH₂); 5.62 (s, 1H, OCH).
c) [3-(2-Aminoethyl)phenoxy]malonsäure-diethylestersulfat
   Man hydriert die nach b) erhaltene Substanz in Ethanol bei 60°C und Normaldruck in Gegenwart von 10proz. Pd auf Kohle, bis die erforderliche H₂-Menge aufgenommen ist. Nach Absaugen des Katalysators wird eingedampft. Man erhält in quantitativer Ausbeute das Sulfat als farbloses Öl.
   ¹H-NMR (300 MHz, d₆-DMSO, ppm):δ = 2.91 (t, J = 7 Hz, 2H, CH₂); 3.11 (t, J = 7 Hz, 2H, CH₂); 5.54 (s, 1H, OCH).
d) <3-[2-(4-Chlor-benzolsulfonylamino)ethyl]phenoxy>malonsäure-diethylester
   Man erhält die Verbindung in einer zu Beispiel 3 b) analogen Verfahrensweise aus [3-(2-Aminoethyl)phenoxy]malonsäure-diethylester-sulfat und 4-Chlorbenzolsulfonylchlorid.
   Ausb. 72 %, Schmp. 60-62°C.
e) <3-[2-(4-Chlor-benzolsulfonylamino)ethyl]phenoxy>malonsäure
   Ein Gemisch aus 12.0 g (25.5 mmol) des nach d) dargestellten Diethylesters, 120 ml 2 N NaOH und 120 ml Methanol wird zwei Stunden bei 50°C gerührt. Dann dampft man i. Vak. das Methanol ab und säuert mit konz. HCl an. Waschen mit eiskaltem Wasser (: die Verbindung ist in Wasser gut löslich) und Trocknen liefert farblose Kristalle.
   Ausb. 7.2 g (68 %). Schmp. 183°C (Zers.).

### Beispiel 11

### 3-[2-(4-Chlor-benzolsulfonamido)ethyl]phenoxyessigsäure-(1H-tetrazol-5-yl)amid

Analog Beispiel 5 erhält man aus 8.39 g (20 mmol) 3-[2-(4-Chlor-benzolsulfonamido)ethyl]phenoxyessigsäure-imidazolid 1.60 g (18 %) der Titelverbindunge in Form farbloser Kristalle, Schmp. 186°C (Methanol)
Das Imidazolid erhält man aus 7.4 g (20 mmol) 3-[2-(4-Chlor-benzolsulfonamido)ethyl]phenoxyessigsäure und 3.25 g (20 mmol) Carbonyldiimidazol in quant. Ausbeute.

### Beispiel 12

### 3-[2-(4-Chlor-benzolsulfonamido)ethyl]phenoxyessigsäure-(1H-tetrazol-5-yl)amid

Zu einer Suspension aus 10.0 g (33,5 mmol) 3-(2-Aminoethyl)phenoxyessigsäure-(1H-tetrazol-5-yl)amid-hydrochlorid (siehe unter f) und 250 ml Wasser gibt man soviel 2N NaOH, daß pH 10 erreicht wird. Dann fügt man 7.3 g (34,6 mmol) 4-Chlor-benzolsulfochlorid zu und sorgt durch kontrollierte Zugabe von 2N NaOH dafür, daß der pH-Wert bis zum Ende der Umsetzung bei pH 10.0 bleibt. Dann tropft man in ein Eis-Salzsäure-Gemisch ein und saugt den ausgefallenen Niederschlag ab. Nach Waschen mit Wasser, Trocknen im Exsikkator und Umkristallisieren aus Eisessig erhält man 12.1 g (82,7 % d.Th.) Produkt mit dem Schmp. 190-199°C (langsame Zersetzung). Das Produkt ist mit dem nach Beispiel 10 erhaltenen identisch.

Die Ausgangsverbindung 3-(2-Aminoethyl)phenoxyessigsäure-(1H-tetrazol-5-yl)amid-hydrochlorid wird auf folgendem Wege erhalten:
a) {3-[2-(Benzyloxycarbonylamino)ethyl]phenyl}-tetrahydropyranyl-ether
   Zu einer Suspension aus 25.0 g (113 mmol) [3-(2-Aminoethyl)phenyl]-tetrahydropyranylether, 200 ml Methylenchlorid und 60 ml 2 N NaOH tropft man unter Kühlen im Eisbad und Rühren langsam eine Lösung aus 118 mmol Chlorameisensäure-benzylester und 50 ml Methylenchlorid. Man rührt anschließend noch eine Std. bei Eisbadtemperatur, danach eine Std. bei Raumtemperatur. Dann trennt man die Phasen. Die CH₂Cl₂-Phase wird zweimal mit Wasser gewaschen, mit Na₂SO₄ getrocknet und schließlich i.Vak. eingedampft. Das Produkt fällt in quantitativer Ausbeute als farbloses Öl an.
b) 3-[2-(Benzyloxycarbonylamino)ethyl]phenol
   Ein Gemisch aus 16.8 g (47,3 mmol) {3-[2-(Benyloxycarbonylamino)ethyl]phenyl}-tetrahydropyranylether, 80 ml Ethanol und 3 g Amberlyst 15 wird zwei Stdn. bei Raumtemperatur gerührt, dann filtriert und eingedampft. Durch Einblasen von Luft unter Anlegen eines leichten Vakuums bei 70°C entfernt man letzte Reste von Ethanol. Man erhält das Produkt in Form eines farblosen Öles.
   Ausb.: quantitativ.
c) 3-[2-Benzyloxycarbonylamino)ethyl]phenoxyessigsäure-ethyl-ester
   Eine Suspension aus 12.79 g (47.1 mmol) 3-[2-(Benzyloxycarbonylamino)ethyl]phenol, 120 ml Butanon-2 und 19.5 g pulv. K₂CO₃ wird eine Std. auf Rückflußtemperatur gehalten, dann kühlt man ab, gibt 200 mg pulv. KJ und 9.12 g (51.9 mmol) Bromessigester zu und rührt 18 Stdn. bei 90°C. Anschließend saugt man ab und wäscht den Filterkuchen mit heißem Butanon-2. Die vereinigten Butanon-Phasen werden eingedampft, den öligen Rückstand nimmt man in Ether auf und extrahiert die Etherphase zweimal mit eiskalter 2N NaOH, danach mit Eiswasser. Nach dem Trocknen mit Na₂SO₄ wird i.Vak. eingedampft. Man erhält 15.62 g (93 % d.Th.) Produkt in Form eines farblosen Öles.
d) 3-[2-(Benzyloxycarbonylamino)ethyl]phenoxyessigsäure
   Ein Gemisch aus 15.55 g (43.5 mmol) 3-[2-(Benzyloxycarbonylamino)ethyl]phenoxy-essigsäure-ethylester, 45 ml Ethanol und 44 ml 2N NaOH wird eine Std. bei 25°C gerührt, dann i.Vak. vom Ethanol befreit. Man verdünnt die zurückbleibende Lösung mit 160 ml Wasser und extrahiert dreimal mit Ether. Danach wird die wäßrige Phase mit conc.HCl angesäuert und das ausgefallene ölige Produkt in Ether aufgenommen. Man trocknet die etherische Lösung (Na₂SO₄) und dampft i.Vak. ein, wobei 13.28 g (93 % d.Th) Säure in Form eines farblosen Öles resultieren.
e) 3-[2-(Benzyloxycarbonylamino)ethyl]phenoxyessigsäure-(1H-tetrazol-5-yl)amid
   Zu einer 40°C warmen Lösung aus 13.1 g (39.8 mmol) 3-[2-(Benzyloxycarbonylamino)ethyl]phenoxyessigsäure und 130 ml abs. THF gibt man 6.48 g (39.9 mmol) Carbonylbisimidazol und rührt anschließend noch 30 min bei 40°C. Dann gibt man 3.39 g (39.9 mmol) wasserfreies 5-Aminotetrazol zu und läßt 18 Stdn. bei 60°C reagieren. Nach Abdestillieren des Tetrahydrofurans erhält man ein farbloses Öl, welches man mit 130 ml 2 N HCl verrührt, wobei Kristallisation eintritt. Man saugt die Kristalle ab, verreibt sie mit verd. HCl, saugt wiederum ab und wäscht mit Wasser. Nach Vakuumtrocknung bei 80°C KOH und Umkristallisieren aus Nitromethan erhält man 12.7 g (50,5 %) Produkt mit dem Schmp. 164-167°C.
f) 3-(2-Aminoethyl)phenoxyessigsäure-(1H-tetrazol-5-yl)amid-hydrochlorid
   Ein Gemisch auf 7.0 g (17,7 mmol) 3-[2-(Benzyloxycarbonylamino)ethyl]phenoxyessigsäure-(1H)-tetrazol-5-yl)amid, 170 ml Methanol, 55 ml Wasser, 18 ml 2N HCl und 0.2 g 10 %ige Palladiumkohle wird 12 Stdn. lang bei 6 bar hydriert. Dann saugt man den Katalysator ab und dampft ein. Der Rückstand wird mit einem Aceton-Ether-Gemisch (1+3 Vol.) verrührt, abgesaugt und getrocknet, Ausb. 4,60 g (86.9 % d.Th.) Hydrochlorid, Schmp. 217-220°C.

### Beispiel 13

In zu Beispiel 12 analoger Weise erhält man die Verbindung 3-[2-(4-Brom-benzolsulfonamido)ethyl]phenoxyessigsäure-(1H-tetrazol-5-yl)amid, Ausb. 91 % d.Th., Schmp. 205-206°C.

### Beispiel 14

### 5-<3-[2-(4-Chlorbenzolsulfonamido)ethyl]phenoxy-methyl>-1H-tetrazol

Man rührt eine Lösung von 1.7 g (4.85 mmol) 3-[2-(4-Chlorbenzolsulfonamido)ethyl]phenoxy-acetonitril, 1.03 g (7.48 mmol) Triethylamin-Hydrochlorid und 0.97 g (14.9 mmol) Natriumamid in 50 ml 1-Methylpyrrolidon 6 Stunden bei 150°C. Dann destilliert man das Lösungsmittel im Hochvakuum ab, nimmt den Rückstand in verd. NaOH auf und schüttelt mit Ether aus. Die wässrige Phase säuert man mit 6 N HCl an, extrahiert mit Essigester und trocknet über Natriumsulfat. Man erhält nach Abziehen des Lösungsmittels 1.19 g (62 %) farblose Kristalle, Schmp. 100-102°C.

Die Vorstufe wird wie folgt gewonnen:
a) 3-[2-(4-Chlor-benzolsulfonamido)ethyl]phenoxyessigsäureamid
   Zu einer Lösung von 29.7 g (72 mmol) 3-[2-(4-Chlorbenzolsulfonamido)ethyl]phenoxy-malonsäure (Darstellung siehe Beispiel 10 e) in 150 ml abs. THF gibt man 14.8 g (72 mmol) Dicyclohexylcarbodiimid, rührt 10 min und leitet ca. eine Stunde lang einen schwachen Ammoniak-Strom ein, laäßt noch 2 Stunden stehen und filtriert. Die Mutterlauge wird eingedampft und der Rückstand aus Isohexan umkristallisiert. Man erhält 18.0 g (68 %) farblose Kristalle, Schmp. 113°C.
b) 3-[2-(4-Chlor-benzolsulfonamido)ethyl]phenoxy-acetonitril
   Man rührt eine Lösung von 15.0 g (40 mmol) des Säureamids und 12.0 g (80 mmol) Phosphorpentoxid in 300 ml Toluol drei Stunden bei 110°C, versetzt den Ansatz mit Wasser und extrahiert mit Essigester. Nach Trocknen über Natriumsulfat und Abziehen des Lösungsmittels erhält man 11.2 g (79 %) farblose Kristalle, Schmp. 92°C.

### Beispiel 15

### 3-[2-(4-Methyl-benzolsulfonamido)ethyl]phenoxy-essigsäure-(1H-tetrazol-5-yl)amid

Die Verbindung erhält man analog Beispiel 10 durch Umsetzung von 4-Methyl-benzolsulfonylchlorid mit [3-(2-Aminoethyl)phenoxy]malonsäurediethylester. Ausb. 65 %, farblose Kristalle, Schmp. 199-201°C.

### Beispiel 16

### 3-[2-(2-Chlor-benzolsulfonamido)ethyl]phenoxy-essigsäure-(1H-tetrazol-5-yl)amid

Die Verbindung erhält man ebenfalls analog Beispiel 10. Ausb. 60 %, farblose Kristalle, Schmp. 150-153°C.

## Patentansprüche

1. Sulfonamide der Formel I 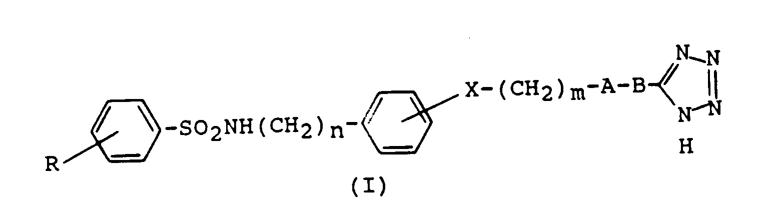 in welcher
R Wasserstoff, ein Halogenatom, eine C₁-C₆-Alkyl-, Trifluormethyl- oder Cyanogruppe bedeuten,
n eine ganze Zahl von 1 bis 3,
m eine ganze Zahl von 0 bis 5 bedeutet,
X eine Bindung, Sauerstoff, eine Carbonylgruppe oder eine Gruppe -CHOH-,
A eine Bindung oder eine Carbonylgruppe,
B eine Bindung oder eine Gruppe -NH- sein kann,
deren physiologisch verträgliche Salze anorganischer oder organischer Basen sowie deren optische Isomere.

2. Verfahren zur Herstellung von Verbindungen der Formel I 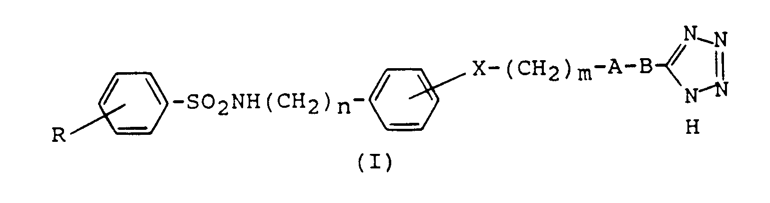 in welcher
R Wasserstoff, ein Halogenatom, eine C₁-C₆-Alkyl-, Trifluormethyl- oder Cyanogruppe bedeuten,
n eine ganze Zahl von 1 bis 3,
m eine ganze Zahl von 0 bis 5 bedeutet,
X eine Bindung, Sauerstoff, eine Carbonylgruppe oder eine Gruppe -CHOH-,
A eine Bindung oder eine Carbonylgruppe,
B eine Bindung oder eine Gruppe -NH- sein kann,
deren physiologisch verträgliche Salze anorganischer oder organischer Basen sowie deren optische Isomere, dadurch gekennzeichnet, daß man in an sich bekannter Weise entweder:
a) eine Verbindung der allgemeinen Formel II 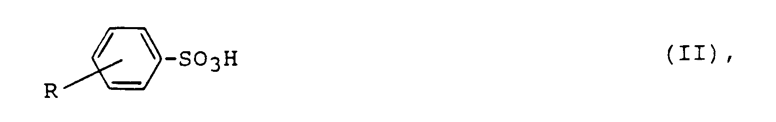 in welcher
R die oben genannte Bedeutung hat, oder ein reaktives Derivat hiervon,
mit einer Verbindung der allgemeinen Formel III 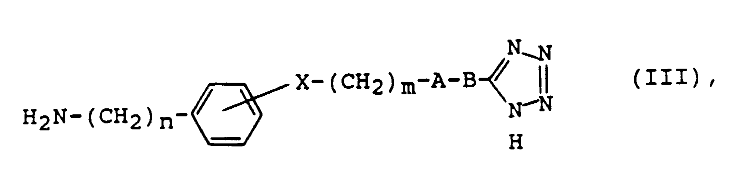 in welcher A, B, X, n und m die oben genannte Bedeutung haben, oder
b) ein Nitril der allgemeinen Formel IV 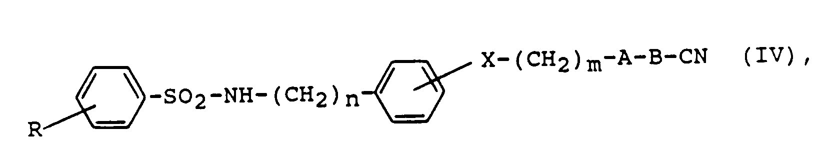 in welcher R, A, B, X, n und m die oben genannte Bedeutung haben,
mit Stickstoffwasserstoffsäure HN₃ oder einem ihrer Salze reagieren läßt,
oder
c) für den Fall, daß A eine CO- und B eine -NH-Gruppe darstellt,
eine Carbonsäure der allgemeinen Formel V 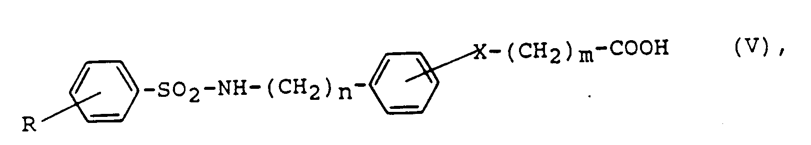 in welcher R, X, n und m die oben genannte Bedeutung haben,
oder ein reaktives Derivat hiervon, wobei hierunter Ester, wie Methyl-, Cyanmethyl-, Ethyl- und p-Nitro phenylester, Anhydride, Amide wie. Imidazolide und Säurehalogenide wie Säurechloride oder Säurebromide verstanden werden,
mit 5-Amino-1,2,3,4-tetrazol VI, gegebenenfalls als Hydrat, 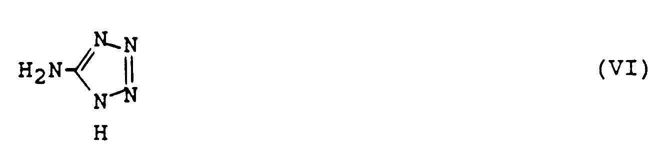 umsetzt,
d) für den Fall, daß A eine CO- und B eine NH-Gruppe darstellt und m die Zahl Eins bedeutet, eine substituierte Malonsäure der allg. Formel VII 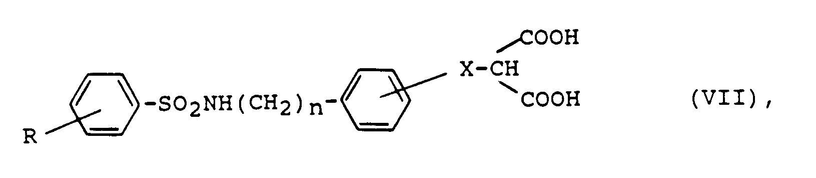 in welcher R und n die oben angegebene Bedeutung haben und X Sauerstoff oder CO symbolisieren,
oder ein reaktives Derivat einer der beiden Carboxylgruppen mit 5-Amino-1,2,3,4-tetrazol VI, ggf. in Form des Hydrats, umsetzt und anschließend die freie Carboxylgruppe unter Decarboxylierungsbedingungen entfernt,
und anschließend gewünschtenfalls Verbindungen der Formel I in andere Verbindungen der Formel I, die erhaltene Verbindung in physiologisch vertragliche Salze überführt und aus Razematen in üblicher Weise die optischen Isomeren isoliert.

3. Arzneimittel, enthaltend eine Verbindung gemäß Anspruch 1 neben üblichen Träger- und Hilfsstoffen.

4. Verwendung von Verbindungen gemäß Anspruch 1 zur Herstellung von Arzneimitteln zur Behandlung von Stoffwechselerkrankungen.

## Claims

1. Sulphonamides of the formula I 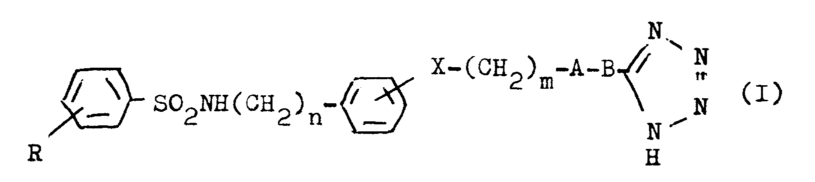 in which R signifies hydrogen, a halogen atom, a C₁-C₆-alkyl, trifluoromethyl or cyano group, n signifies a whole number from 1 to 3, m a whole number from 0 to 5, X can be a bond , oxygen, a carbonyl group or a group -CHOH-, A a bond or a carbonyl group, B a bond or a group -NH-, their physiologically compatible salts of inorganic or organic bases, as well as their optical isomers.

2. Process for the preparation of compounds of the formula I 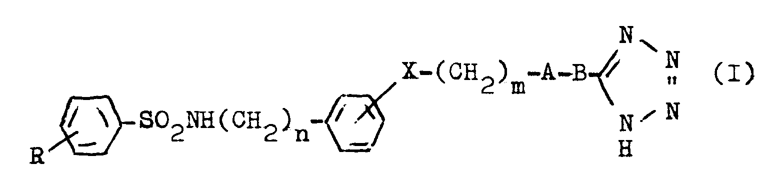 in which R signifies hydrogen, a halogen atom, a C₁-C₆-alkyl, trifluoromethyl or cyano group, n signifies a whole number from 1 to 3, m a whole number from 0 to 5, X can be a bond, oxygen, a carbonyl group or a group -CHOH-, A a bond or a carbonyl group, B a bond or a group -NH-, of their physiologically compatible salts inorganic or organic bases, as well as their optical isomers, characterised in that, in per se known manner, one either
a) allows a compound of the general formula II 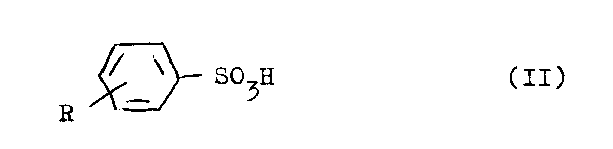 in which R has the above-given meaning, or a reactive derivative thereof, to react with a compound of the general formula III 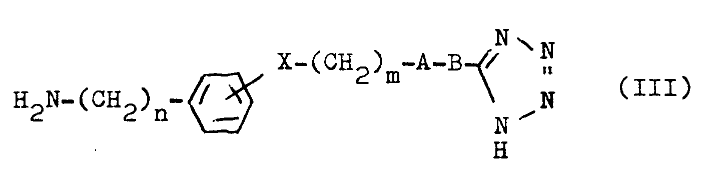 in which A, B, X, n and m have the above-given meaning; or
b) a nitrile of the general formula IV 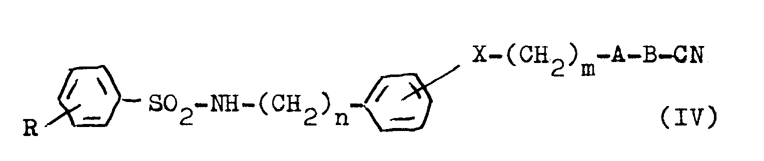 in which R, A, B, X, n and m have the above-given meanings, with hydrazoic acid HN₃ or one of its salts; or
c) for the case that A represents a -CO- and B an -NH- group, reacts a carboxylic acid of the general formula V 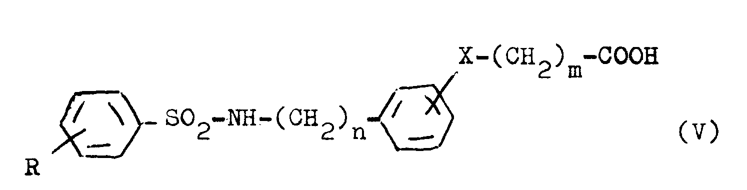 in which R, X, n and m have the above-given meaning, or a reactive derivative thereof, whereby hereunder are understood eaters, such as methyl, cyanomethyl, ethyl and p-nitrophenyl eaters, anhydrides, amides, such as imidazolide, and acid halides, such as acid chlorides or acid bromides, with 5-amino-1,2,3,4-tetrazole VI, possibly as hydrate: 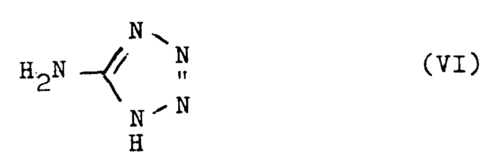 or
d) for the case that A represents a -CO- and B an -NH- group and m signifies the number one, reacts a substituted malonic acid of the formula VII 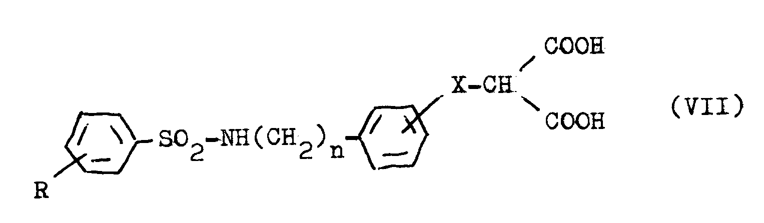 in which R and n have the above-given meaning and X symbolises oxygen or CO, or a reactive derivative of one of the two carboxyl groups, with 5-amino-1,2,3,4-tetrazole VI, possibly in the form of the hydrate, and subsequently removes the free carboxyl group under decarboxylation conditions;
and subsequently, if desired, converts compounds of the formula I into other compounds of the formula I, the compound obtained into physiologically compatible salts and isolates the optical isomers from racemates in the usual way.

3. Medicaments containing a compound according to claim 1, besides usual carrier and adjuvant materials.

4. Use of compounds according to claim 1 for the preparation of medicaments for the treatment of metabolic diseases.

## Revendications

1. Sulfonamides de formule I 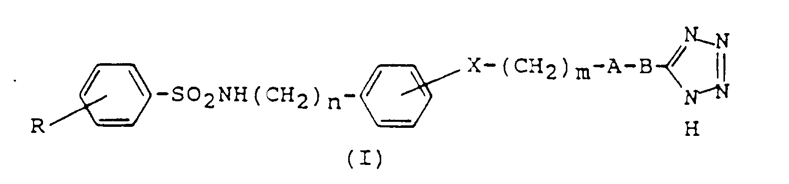 dans laquelle
R représente un atome d'hydrogène, un atome d'halogène, un groupe alkyle(C₁-C₆), un groupe trifluorométhyle ou un groupe cyano,
n vaut un nombre entier de 1 à 3,
m vaut un nombre entier de 0 à 5,
X représente une liaison, un atome d'oxygène, un groupe carbonyle ou un groupe -CHOH-,
A représente une liaison ou un groupe carbonyle,
B représente une liaison ou un groupe -NH-,
leurs sels physiologiquement acceptables, formés avec des bases organiques ou inorganiques, ainsi que leurs isomères optiques.

2. Procédé de préparation de composés de formule I 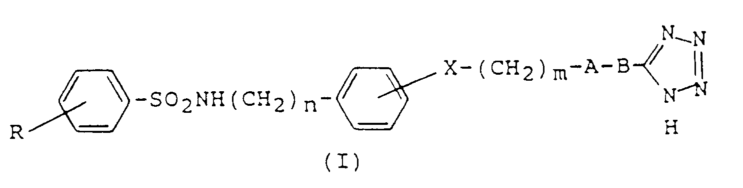 dans laquelle
R représente un atome d'hydrogène, un atome d'halogène, un groupe alkyle(C₁-C₆), un groupe trifluorométhyle ou un groupe cyano,
n vaut un nombre entier de 1 à 3,
m vaut un nombre entier de 0 à 5,
X représente une liaison, un atome d'oxygène, un groupe carbonyle ou un groupe -CHOH-,
A représente une liaison ou un groupe carbonyle,
B représente une liaison ou un groupe -NH-,
leurs sels physiologiquement acceptables, formés avec des bases organiques ou inorganiques, ainsi que leurs isomères optiques, caractérisé en ce que, de manière connue en soi, soit
a) on fait réagir un composé de formule générale II 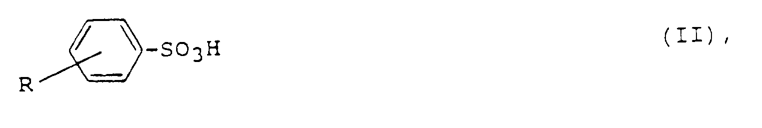 dans laquelle R a la signification mentionnée, ou un de ses dérivés réactifs,
avec un composé de formule générale III 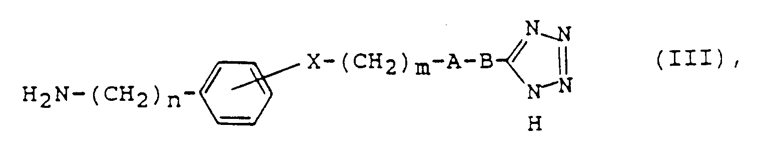 dans laquelle A, B, X, n et m ont les significations mentionnées ci-dessus, soit
b) on fait réagir un nitrile de formule générale IV 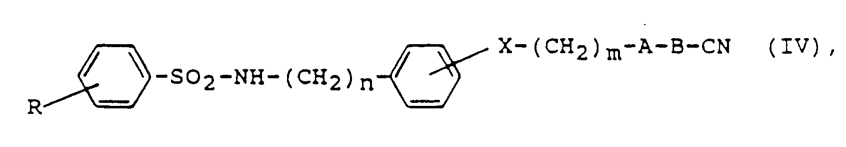 dans laquelle R, A, B, X, n et m ont les significations mentionnées ci-dessus,
avec l'acide hydroazoïque HN₃ ou un de ses sels, soit
c) dans le cas où A représente un groupe CO et B un groupe -NH-, on fait réagir un acide carboxylique de formule générale V 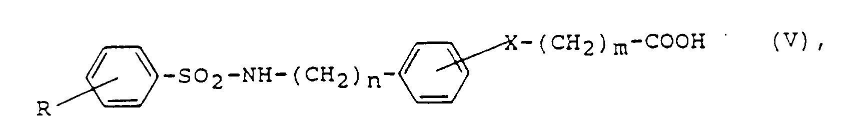 dans laquelle R, X, n et m ont les significations mentionnées ci-dessus,
ou un de ses dérivés réactifs, qui peut être un ester tel qu'un ester de méthyle, de cyanométhyle, d'éthyle ou de p-nitrophényle, un anhydride, un amide tel qu'un imidazolide et un halogénure d'acide tel qu'un chlorure d'acide ou un bromure d'acide,
avec un 5-amino-1,2,3,4-tétrazole VI, éventuellement sous forme d'hydrate, 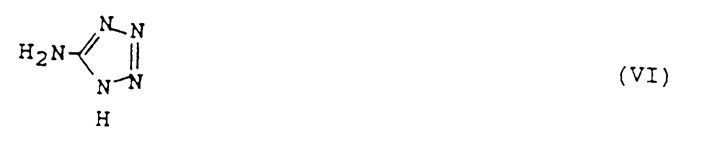 soit
d) dans le cas où A représente un groupe CO et B un groupe NH, et m vaut un, on fait réagir un acide malonique substitué, de formule générale VII 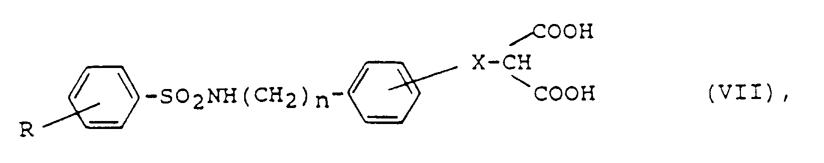 dans laquelle R et n on les significations mentionnées ci-dessus et X représente un atome d'oxygène ou un groupe CO,
ou un des dérivés réactifs d'un des groupes carboxyle ou des deux, avec le 5-amino-1,2,3,4-tétrazole VI, éventuellement sous la forme d'hydrate, et ensuite, on élimine le groupe carboxyle libre dans des conditions de décarboxylation,
et ensuite, si on le souhaite, on transforme les composés de formule I en d'autres composés de formule I, on transforme les composés obtenus en leurs sels physiologiquement acceptables, et à partir des racémates, on isole les isomères optiques de manière usuelle.

3. Médicament, contenant un composé selon la revendication 1, en plus de véhicules et adjuvants usuels.

4. Utilisation de composés selon la revendication 1, pour la préparation de médicaments destinés au traitement des troubles du métabolisme.
